# FASCICULE DE BREVET EUROPEEN

(11) **EP 2 274 360 B1**
(45) Date de publication et mention de la délivrance du brevet: **31.07.2013**
(21) Numéro de dépôt: 09745908.5
(22) Date de dépôt: 29.04.2009
(51) Int. Cl.: C08G 63/08, C08G 63/87, C08G 63/82

(54) **NOUVEAUX SYSTÈMES CATALYTIQUES POUR LA (CO)POLYMÉRISATION DE LACTONES PAR OUVERTURE DE CYCLE**
NEUARTIGE KATALYSATORSYSTEME ZUR RINGÖFFNENDEN (CO)POLYMERISIERUNG VON LACTONEN
NOVEL CATALYTIC SYSTEMS FOR THE RING-OPENING (CO)POLYMERIZATION OF LACTONES

(30) Priorité: 30.04.2008 FR 0802437
(43) Date de publication de la demande: 19.01.2011
(73) Titulaire: IPSEN PHARMA S.A.S., 91200 Boulogne-Billancourt (FR)
(72) Inventeur: BOURISSOU, Didier, F-31830 Plaisancedu Touch (FR); MARTIN-VACA, Blanca, F-31100 Toulouse (FR); ALBA, Aurélie, F-31400 Toulouse (FR); CHERIF-CHEIKH, Roland, E-08860 Castelldefels (Barcelona) (ES); DE SOUSA DELGADO, Anne-Paula, E-08750 Molins de Rei (Barcelona) (ES)
(74) Mandataire: Audonnet, Nathalie
(86) Numéro de dépôt international: PCT/FR2009/000503
(87) Numéro de publication internationale: WO 2009/138589

(56) Documents cités:
- WO-A-2004/052980
- US-A- 3 655 631
- EAGLE C.T., KAVALLIERATOS K., BRYAN J.C.: "Nonpreorganized neutral acyclic anion receptors: Structural investigations of N,N'-diphenyl-1,3-benzenedisulfonamide and N,N'-bis(4-t-butylphenyl)-1,3-benzenedisul fonamide" JOURNAL OF CHEMICAL CRYSTALLOGRAPHY, vol. 32, no. 7, 2002, pages 165-170, XP002504641

## Description

La présente invention concerne l'utilisation d'un système constitué d'une base et d'un sulfonamide, en tant que catalyseur de (co)polymérisation de lactones par ouverture de cycle. La présente invention concerne également de nouveaux sulfonamides et un procédé de (co)polymérisation de lactones par ouverture de cycle comprenant l'utilisation de sulfonamides en association avec une base comme système catalytique.

De nos jours, une attention croissante est portée aux polymères synthétiques pour l'élaboration d'organes artificiels et la formulation de médicaments [Chem. Eng. News 2001, 79 (6), 30]. Les polymères concernés doivent respecter un certain nombre de critères et, en particulier, ils doivent être biocompatibles. Le caractère biodégradable est un avantage supplémentaire si le polymère doit être éliminé après une période appropriée d'implantation dans un organisme. A cet égard, les copolymères à base d'acide lactique et glycolique (PLGA) présentent un très grand intérêt car ils sont sensibles à l'hydrolyse et sont dégradés *in vivo* avec libération de sous-produits non-toxiques. Le champ d'application des PLGA est très vaste (Adv. Mater. 1996, 8, 305 et Chemosphere 2001, 43, 49). Dans le domaine chirurgical, ils sont utilisés pour la synthèse de fils multi-brins, de sutures, d'implants, de prothèses... En pharmacologie, ils permettent l'encapsulation, le transfert et la libération contrôlée de principes actifs.

Pour toutes ces applications, le facteur clé est la vitesse de dégradation des PLGA qui dépend bien sûr de leur structure (longueur de chaîne, dispersité, proportion, stéréochimie et enchaînement des monomères...). Ces dernières années, de nombreux travaux ont donc été consacrés à la mise au point de catalyseurs et/ou amorceurs de (co)polymérisation, c'est-à-dire de polymérisation ou de copolymérisation, du lactide et/ou du glycolide permettant de préparer des PLGA de structure contrôlée.

L'utilisation de systèmes métalliques conduit le plus souvent à une contamination des copolymères ainsi obtenus par la présence de sels métalliques, ce qui constitue parfois une limitation importante selon les applications envisagées. La mise au point de systèmes non-métalliques permettant la (co)polymérisation contrôlée du lactide et/ou du glycolide constitue donc un enjeu majeur. La présente invention s'inscrit dans ce cadre.

La déposante propose donc l'utilisation d'un système catalytique simple, constitué d'un catalyseur et d'un additif de (co)polymérisation, et qui permet de contrôler la longueur de chaîne mais également la nature des extrémités de chaîne des (co)polymères préparés.

La présente invention a donc pour objet l'utilisation d'un sulfonamide en association avec une base comme système catalytique de (co)polymérisation de lactones par ouverture de cycle.

Le terme (co)polymérisation signifie polymérisation ou copolymérisation. Ce terme inclut le terme (co)oligomérisation, qui signifie oligomérisation ou cooligomérisation avec des degrés de polymérisation (DP) inférieurs à 30. Ainsi, par exemple, la (co)polymérisation du lactide et du glycolide couvre la polymérisation du lactide, la polymérisation du glycolide mais également la copolymérisation du lactide et du glycolide. Le terme (co)polymère signifie polymère ou copolymère. Ce terme inclut le terme (co)oligomère, qui signifie oligomère ou cooligomère avec des degrés de polymérisation (DP) inférieurs à 30. Ainsi, par exemple, un (co)polymère du lactide et du glycolide couvre un polymère du lactide, un polymère du glycolide mais également un copolymère du lactide et du glycolide.

Selon la présente invention, le terme sulfonamide représente une molécule comportant au moins une fonction -SO₂-NH-, telle qu'un monosulfonamide ou un bisulfonamide. Le terme monosulfonamide représente une molécule comportant une fonction -SO₂-NH-, et le terme bisulfonamide représente une molécule comportant deux fonctions - SO₂-NH-.

L'invention a plus particulièrement pour objet l'utilisation d'un sulfonamide tel que défini ci-dessus pour la (co)polymérisation de dilactones.

L'invention a également pour objet l'utilisation d'un sulfonamide tel que défini ci-dessus pour la copolymérisation du lactide et/ou du glycolide, et préférentiellement pour la polymérisation du lactide.

Selon la présente invention, la base utilisée est préférentiellement une amine tertiaire ;
et plus particulièrement, une amine tertiaire choisie parmi :
- diisopropyléthylamine ;
- spartéine ;
- N,N-diméthylcyclohexylamine ;
- N,N,N"N"-tétraméthyl-1,2-cyclohexanediamine ; et
- 4-diméthylaminopyridine.

Selon une variante de l'invention, le sulfonamide utilisé est un monosulfonamide,
et de préférence un monosulfonamide de formule générale (**I**) : 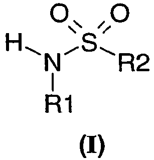 sous forme racémique, d'énantiomère ou toute combinaison de ces formes, dans laquelle :
R1 et R2 représentent indépendamment un radical alkyle, haloalkyle ou aryle éventuellement substitué.

Au sens de la présente invention, les radicaux aryles peuvent être de type mono ou polycycliques aromatiques. Les radicaux aryles monocycliques peuvent être choisis parmi les radicaux phényles, tolyles, xylyles, mésityles, cuményles et de préférence phényles. Les radicaux aryles polycycliques peuvent être choisis parmi les radicaux naphtyle, anthryle, phénanthryle, fluorényle. Ils peuvent être optionnellement substitués par un ou plusieurs radicaux tels que alkyle, haloalkyle, alkoxy, alkoxycarbonyle, alkylcarbonyloxy, halo, cyano, nitro, aryle, aryloxy, aryloxycarbonyl, arylcarbonyloxy.

L'expression halo signifie fluoro, chloro, bromo ou iodo, et de préférence fluoro.

L'expression alkyle représente un radical alkyle de 1 à 8 atomes de carbone. Cette expression couvre les radicaux alkyle ayant de 1 à 6 atomes de carbone linéaires ou ramifiés et en particulier les radicaux alkyle ayant de 1 à 4 atomes de carbone tels que les radicaux méthyle, éthyle, propyle, isopropyle, butyle, isobutyle, sec butyle et tert butyle, et de préférence méthyle. L'expression couvre également les radicaux comprenant plus de 6 atomes de carbone tels que les radicaux heptyle et octyle.

Le terme haloalkyle représente un radical alkyle tel que défini ci-dessus substitué par un ou plusieurs groupes halo indentiques ou différents tels que définis ci-dessus, comme par exemple trifluorométhyle, 1,2-dichloroéthyle et de préférence trifluorométhyle.

Le terme aryloxy désigne les radicaux dans lesquels le radical aryle est tel que défini ci-dessus comme par exemple les radicaux phényloxy, tolyloxy, naphtyloxy, anthryloxy et phénanthryloxy. Le terme aryloxycarbonyle désigne de préférence les radicaux dans lesquels le radical aryloxy est tel que défini ci-dessus, comme par exemple phényloxycarbonyle, tolyloxycarbonyle. Le terme arylcarbonyloxy désigne de préférence les radicaux dans lesquels le radical aryl est tel que défini ci-dessus, comme par exemple phénylcarbonyloxy, tolylcarbonyloxy ou naphtylcarbonyloxy.

Le terme alkoxy désigne les radicaux dans lesquels le radical alkyle est un radical de 1 à 8 atomes de carbone tel que défini ci-dessus comme par exemple les radicaux méthoxy, éthoxy, propyloxy ou isopropyloxy mais également butoxy linéaire, secondaire ou tertiaire, pentyloxy.

Le terme alkoxycarbonyle désigne de préférence les radicaux de type alkyl-O-C(O)-dans lesquels le radical alkyl est tel que défini ci-dessus comme par exemple méthoxycarbonyle, éthoxycarbonyle.

Le terme alkylcarbonyloxy désigne de préférence les radicaux de type alkyl-C(O)-O-dans lesquels le radical alkyl est tel que défini ci-dessus comme par exemple méthylcarbonyloxy, éthylcarbonyloxy.

L'invention a également préférentiellement pour objet l'utilisation d'un monosulfonamide de formule générale (I), dans laquelle R1 et R2 représentent indépendamment un radical phényle, alkyle ou haloalkyle.

L'invention a plus particulièrement pour objet l'utilisation d'un sulfonamide tel que défini ci-dessus choisi parmi: 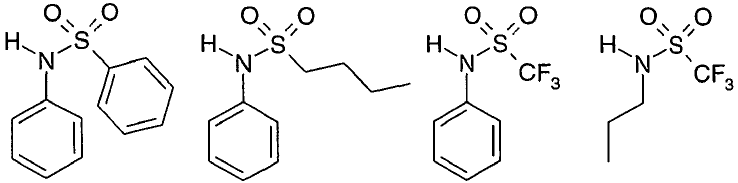 Selon une autre variante de l'invention, le sulfonamide utilisé est un bisulfonamide, et de préférence un bisulfonamide de formule générale (**IIa**) ou (**IIb**) 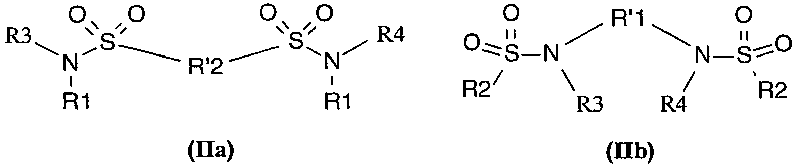 sous forme racémique, d'énantiomère ou toute combinaison de ces formes, dans laquelle :
R1 et R2 représentent un radical alkyle, haloalkyle ou aryle éventuellement substitué ;
R'1 et R'2 représentent un radical arylène, alkylène, cycloalkylène, tous ces radicaux étant éventuellement substitués ;
R3 et R4 représentent indépendamment un atome d'hydrogène ou un radical alkyle, préférentiellement R3 et R4 représentent indépendamment un atome d'hydrogène ou un radical méthyle et très préférentiellement R3 et R4 représentent un atome d'hydrogène.

Au sens de la présente invention, le terme arylène représente un groupe aryle divalent, le groupe aryle étant tel que défini précédemment, le terme alkylène représente un groupe alkyle divalent, le groupe alkyle étant tel que défini précédemment et le terme cycloalkylène désigne un groupe cycloalkyle divalent, le groupe cycloalkyle étant tel que défini ci-dessous.

Les radicaux cycloalkyles sont choisis parmi les cycloalkyles monocycliques saturés ou insaturés. Les radicaux cycloalkyles monocycliques saturés peuvent être choisis parmi les radicaux ayant de 3 à 7 atomes de carbone tels que les radicaux cyclopropyle, cyclobutyle, cyclopentyle, cyclohexyle ou cycloheptyle. Les radicaux cycloalkyles insaturés peuvent être choisis parmi les radicaux cyclobutène, cyclopentène, cyclohexène, cyclopentadiène, cyclohexadiène.

De manière très préférentielle, R1 et R2 représentent un radical phényle éventuellement substitué, alkyle ou haloalkyle ; R'1 représente un radical cycloalkylène, alkylène éventuellement substitué par un radical phényle ; R'2 représente un radical alkylène ou phénylène.

Plus particulièrement, R1 et R2 représentent un radical alkyle, trifluorométhyle ou un radical phényle éventuellement substitué par méthyle ou trifluorométhyle.

L'invention a plus particulièrement pour objet l'utilisation d'un sulfonamide choisi parmi : 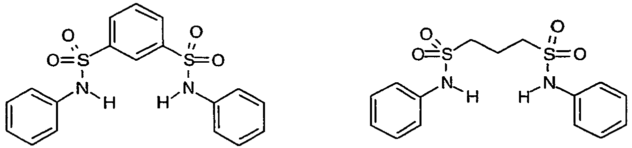 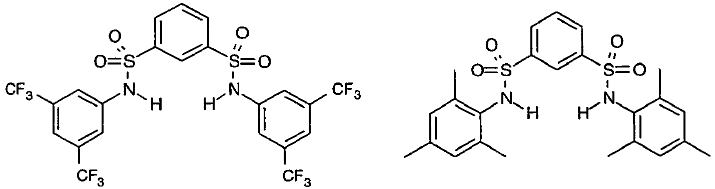 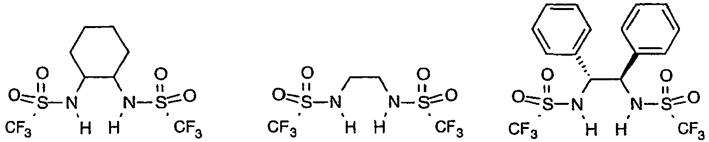 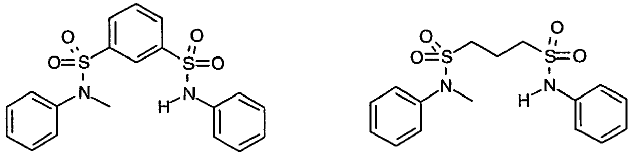 et encore plus particulièrement choisi parmi :

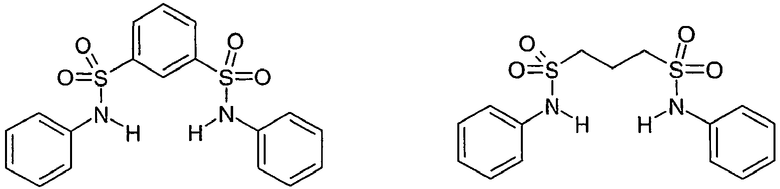

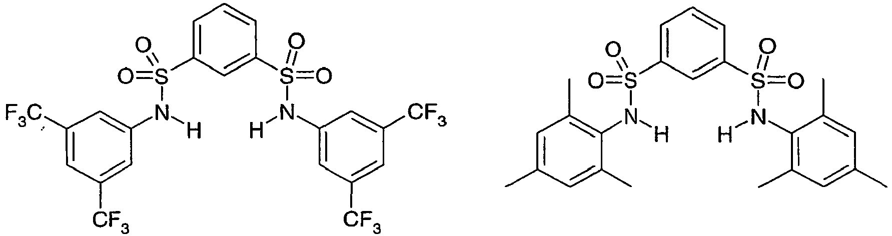

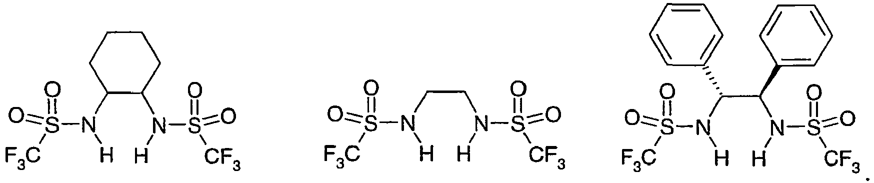

De manière très préférentielle, la base utilisée pour la présente invention est la 4-diméthylaminopyridine.

L'invention a également pour objet les composés suivants : 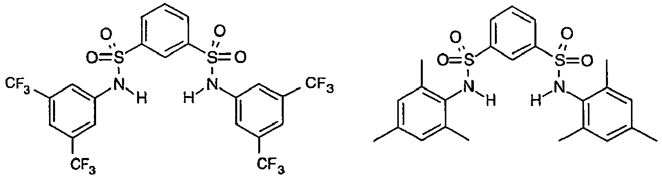 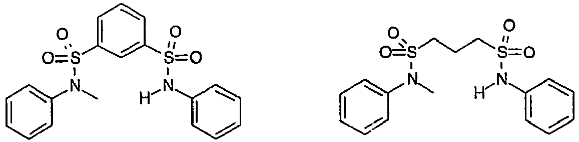 en tant que sulfonamides tels que définis ci-dessus, et plus préférentiellement, l'invention a également pour objet les composés suivants :

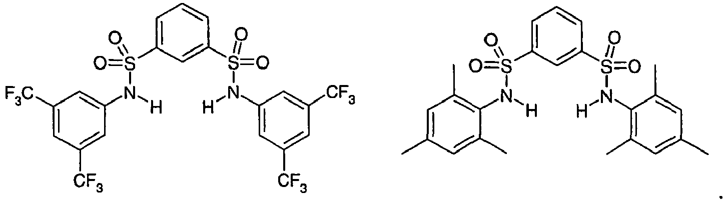

L'invention a plus particulièrement pour objet également un procédé de (co)polymérisation par ouverture de cycle caractérisé en ce qu'il fait intervenir l'utilisation d'un sulfonamide en association avec une base comme catalyseur, telle que définie précédemment.

Préférentiellement, ce procédé utilise un solvant de (co)polymérisation, à une température comprise entre 0° C et 250° C (plus préférentiellement entre la température ambiante et 150° C), pendant une durée comprise entre quelques minutes et 300 heures (plus préférentiellement entre une heure et soixante-douze heures). La température est choisie en fonction du solvant de telle manière à ce qu'elle soit dans la gamme ci-dessus et au maximum la température d'ébullition du solvant si cette température est inférieure à 250° C.

Très préférentiellement, ce procédé fait intervenir le lactide et / ou le glycolide en tant que monomère.

La présente invention présente de nombreux avantages, en particulier :
- les systèmes catalytiques sont facilement accessibles et bon marché ;
- les sulfonamides sont synthétisés de manière simple et avec de bons rendements ;
- les sulfonamides ont des structures diverses, permettant d'envisager de nombreux systèmes catalytiques différents ;
- les sulfonamides ont des structures stables à l'air à température ambiante ;
- la distribution de masse des polymères obtenus est très étroite ; les indices de polydispersité obtenus grâce à la présente invention sont en effet compris entre 1,05 et 1,20 ;
- la (co)polymérisation par ouverture de cycle catalysée par les sulfonamides est reproductible ;
- la (co)polymérisation par ouverture de cycle catalysée par les sulfonamides peut être mise en oeuvre dans des solvants divers tels que le dichlorométhane, le toluène ou le tétrahydrofurane.

L'invention concerne enfin des polymères ou copolymères du lactide et/ou du glycolide obtenus ou susceptibles d'être obtenus par la mise en oeuvre d'un procédé tel que décrit ci-dessus.

A moins qu'ils ne soient définis d'une autre manière, tous les termes techniques et scientifiques utilisés dans la présente demande ont la même signification que celle couramment comprise par un spécialiste ordinaire du domaine auquel appartient l'invention.

Les catalyseurs préparés comportent tous une ou deux fonctions -SO₂NH- : on parle alors respectivement de monosulfonamide et de bisulfonamide.

La partie expérimentale suivante est présentée pour illustrer les procédures ci-dessus et ne doit en aucun cas être considérée comme une limite à la portée de l'invention.

### PARTIE EXPERIMENTALE

Les produits ont été caractérisés selon les méthodes classiques connues de l'homme de l'art décrites ci-dessous.

Les spectres RMN du ¹H, du ¹³C, et du ¹⁹F sont réalisés sur des spectromètres Bruker Avance 300. Les déplacements sont comptés positivement vers les champs forts, et exprimés en ppm. Les références sont le tétraméthylsilane pour le ¹H et le ¹³C, et l'acide trifluoroacétique pour le ¹⁹F. Les abréviations suivantes ont été utilisées pour décrire les signaux : s (singulet), br s (singulet large), d (doublet), t (triplet), q (quadruplet), qt (quintuplet), dd (doublet dédoublé), m (multiplet).

Les points de fusion ont été mesurés sur un appareil digital Electrothermal.

Les spectres de masse ont été réalisés en utilisant les méthodes d'ionisation chimique (IC) ou d'impact électronique (IE) sur des spectromètres Thermo TSQ 700, Applied Biosystem API-365 ou Applied Biosystem Qtrap. Les analyses à haute résolution (HRMS) ont été effectuées sur un appareil Micromass Waters LCT.

Les masses moyennes en nombre (Mn) et en masse des polymères (Mw), ainsi que les indices de polydispersité (Ip), sont déterminés par chromatographie d'exclusion stérique sur une chaîne HPLC Waters 712 (dans le THF, 1 mL/min, T = 35° C, colonne Styragel HR1 ou Styragel HR4E, calibration avec des standards polystyrène).

### Préparation 1 : Préparation des sulfonamides à partir de chlorure de sulfonyle (exemples 1 à 6)

Une première famille est obtenue à partir d'un squelette comportant une ou deux fonctions chlorure de sulfonyle, sur laquelle est greffée l'amine choisie. Ces catalyseurs, stables à l'air et à température ambiante, ont été entièrement caractérisés (RMN ¹H, ¹³C, SM, analyses élémentaires, point de fusion).

### Préparation 1a : Préparation de monosulfonamides

Les monosulfonamides ont été préparés de la manière suivante :

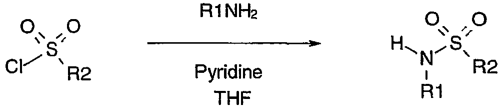

A un mélange d'amine R1NH₂ (1 équivalent) et de pyridine (1 équivalent) dans le tétrahydrofurane (1,8 mol.L⁻¹) est lentement ajouté le chlorure de sulfonyle R2SO₂Cl (1 équivalent) et le milieu réactionnel est agité à température ambiante, c'est-à-dire à une température comprise entre 18° C et 30° C, jusqu'à conversion totale des réactifs, l'avancement de la réaction étant suivi par RMN ¹H. Le solvant est ensuite évaporé sous vide et le brut obtenu est purifié par chromatographie sur colonne de silice, la phase mobile étant un gradient CH₂Cl₂/MeOH.

Les monosulfonamides suivants ont ainsi été préparés :

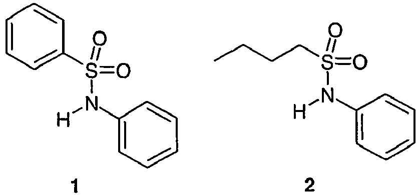

### Exemple 1 :

**RMN ¹H** (CDCl_{3,} 300 MHz): δ 7,03 (s, 1H, NH) ; 7,23-7,31 (m, 3H, CH); 7,39-7,43 (m, 2H, CH) ; 7,58-7,63 (m, 2H, CH) ; 7,68-7,73 (m, 1H, CH) ; 7,96 (d, ³J = 7,7 Hz, 2H, CH) ppm; ¹³C (CDCl₃, 75 MHz) : δ 121,7 (CH); 125,5 (CH) ; 127,3 (CH) ; 129,1(CH); 129,4 (CH) ; 133,1 (CH) ; 136,4 (C), 138,96 (C) ppm ; **SM (IE) :** 233 [M]⁺; **Analyse élémentaire :** *Calculé pour (C₁₂H₁₁NO₂S)* C 61,78 %, H 4,75 %, N 6,00 %. *Mesuré* C 61,86 %, H 4,50 %, N 5,97 %. Point de fusion : 110,5-112,0° C.

### Exemple 2 :

**RMN ¹H** (CDCl₃, 300 MHz) : δ 0,90 (t, ³J = 7,2 Hz, 3H, CH₃) ; 1,36-1,48 (m, 2H, CH₂); 1,75-1,86 (m, 2H, CH₂); 3,06-3,12 (m, 2H, CH₂) ; 6,31 (s, 1H, NH) ; 7,15-7,21 (m, 3H, CH) ; 7,32-7,38 (m, 2H, CH) ppm ; ¹³C (CDCl₃, 75 MHz) : δ 13,4(CH₃); 21,3 (CH₂) ; 25,2 (CH₂) ; 51,1 (CH₂) ; 120,3 (CH) ; 124,8 (CH) ; 129,5 (CH) ; 137,1 (C) ppm ; **SM (IE)** : 213 [M]⁺; **Analyse élémentaire :** *Calculé pour (C₁₀H₁₅N₂S)* C 56,31 %, H 7,09 %, N 6,57 %. *Mesuré* C 55,72 %, H 7,27 %, N 6,42 %.

### Préparation 1b : Préparation de bisulfonamides

Les bisulfonamides ont été préparés selon le mode opératoire suivant :

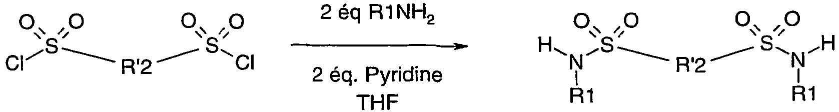

A un mélange d'amine R1NH₂ (2 équivalents) et de pyridine (2 équivalents) dans le tétrahydrofurane (1 mol.L⁻¹) est ajouté le chlorure de sulfonyle (1 équivalent). Le milieu réactionnel est agité à température ambiante jusqu'à conversion totale des réactifs, l'avancement de la réaction étant suivi par RMN ¹H. Le solvant est ensuite évaporé sous vide et le brut obtenu est purifié par chromatographie sur colonne de silice, la phase mobile étant un gradient CH₂Cl₂/MeOH.

Les bisulfonamides suivants ont ainsi été préparés :

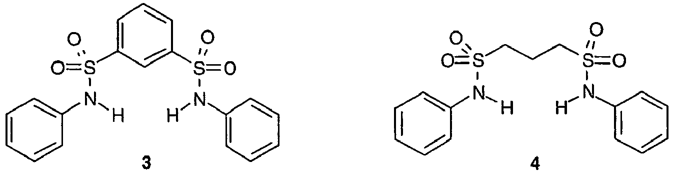

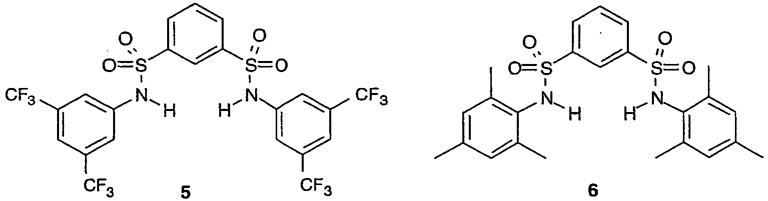

### Exemple 3 :

**RMN ¹H** (CD₃OD, 300 MHz) : δ 6,97-7,21 (m, 10H, GH) ; 7,55 (t, ³J = 7,8 Hz, 1H, CH) ; 7,86 (dd, 2H, ⁴J = 1,5 Hz et ³J = 7,8 Hz, 2H, CH) ; 8,16 (t, ⁴J = 1,5 Hz, 1H, CH) ppm ; ¹³C (CD₃OD, 75 MHz) : δ 122,6 (CH) ; 126,2 (CH) ; 127,0 (CH) ; 130,3 (CH) ; 131,0 (CH) ; 132,0 (CH) ; 138,3 (C) ; 142,3 (C) ppm ; **SM (IE)** : 388 [M]⁺; **Analyse élémentaire :** *Calculé pour (C₁₈H₁₄N₂O₄S₂)* C 55,65 %, H 5,15 %, N 7,21 %. *Mesuré* C 56,13 %, H 3,82 %, N 7,21 % ; **Point de fusion :** 157° C.

### Exemple 4 :

**RMN ¹H** (CDCl_{3,} 300 MHz) : δ 2,36-2,41 (qt, ³J = 7,2 Hz, 2H, CH₂) ; 3,27-3,32 (t, ³J = 7,2 Hz, 4H, CH₂) ; 7,04 (s, 2H, NH) ; 7,18-7,23 (m, 4H, CH) ; 7,27 (m, 2H, CH) ; 7.35 (m, 4H, CH) ppm ; ¹³C (CDCl₃, 75 MHz) : δ 18,1 (CH₂) ; 48,8 (CH₂) ; 121,2 (CH) ; 125,7 (CH) ; 129,8 (CH) ; 136,4 (C) ppm ; **SM (IE)** : 354 [M]⁺; **Analyse élémentaire :** *Calculé pour (C₁₅H₁₈N₂O₄S₂)* C 50,83 %, H 5,12 %, N 7,90 %. *Mesuré* C 51,01 %, H 4,74 %, N 7,85 %. **Point de fusion :** 129,8-131,6° C.

### Exemple 5 :

**RMN ¹H** (CD₃OD, 300 MHz) : δ 7,58 (s, 4H, CH) ; 7,60 (s, 2H, CH) ; 7,74 (t, 1H, ³J = 1,7 Hz, CH) ; 8,03 (dd, ⁴J = 1,7 Hz, ³J = 7,7 Hz, 2H, CH) ; 8,21 (t, ³J = 7,7 Hz, 1H, CH) ppm ; ¹³C (CD₃OD, 75 MHz) : δ 118,7 (q, J_{CF} = 3,9 Hz, CH) ; 120,9 (q, J_{CF} = 3,5 Hz, CH) ; 124,2 (q, J_{CF} = 271,6 Hz, CF₃) ; 126,6 (C) ; 132,1 (CH) ; 132,5 (CH) ; 133,9 (q, J_{CF} = 33,7 Hz, C) ; 140,6 (C) ; 142,2 (C) ppm ; **¹⁹F** (CD₃OD, 280 MHz) : δ -63,2 ppm ; **SM (IC) :** 678 [M+NH4]⁺ ; **Analyse élémentaire :** *Calculé pour (C₂₂H₁₂F₁₂N₂O₄S₂)* C 40,01 %, H 1,83 %, N 4,24 %. *Mesuré* C 40,38 %, H 1,26 %, N 4,19 %; **Point de fusion :** 159,0-159,6° C.

### Exemple 6 :

**RMN ¹H** (CDCl₃, 300 MHz) : δ 2,00 (s, 12H, CH₃) ; 2,25 (s, 6H, CH₃) ; 6,71 (s, 2H, NH) ; 6,84 (s, 4H, CH) ; 7,51 (t, ³J = 7,8 Hz, 1H, CH) ; 7,80-7,83 (q, ⁴J = 1,7 Hz, ³J = 7,8 Hz, 2H, CH) ; 8,58 (t, ⁴J = 1,7 Hz, 1H, CH) ppm ; ¹³C (CDCl_{3,} 75 MHz) : δ 18,7 (CH₃) ; 20,9 (CH₃) ; 125,8 (CH); 129,3 (C); 129,7 (CH) ; 130,0 (CH) ; 131,1 (CH) ; 137,5 (C) ; 138,1 (C); 142,6 (C) ppm ; **SM (IE)** : 233 [M]⁺; **Analyse élémentaire :** *Calculé pour (C₂₄H₂₈N₂O₄S₂)* C 60,99 %, H 5,97 %, N 5,93 %. *Mesuré* C 60,90 %, H 5,91 %, N 5,85 %. **Point de fusion :** 197,9-199,5° C.

### Préparation 2 : Préparation des sulfonamides à partir de l'anhydride sulfonique (exemples 7 à 11)

Une seconde famille de sulfonamide a également été synthétisée à partir des amines, par réaction avec l'anhydride trifluorométhanesulfonique.

Les catalyseurs préparés ainsi ont été caractérisés. Ils sont stables à l'air et à température ambiante.

### Préparation 2a : Préparation de monosulfonamides

Les monosulfonamides ont été préparés selon le mode opératoire suivant :

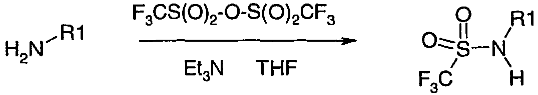

A un mélange d'amine (1 équivalent, 3 mol.L⁻¹) et de triéthylamine (1,1 équivalents) dans le dichlorométhane anhydre à 0° C, est ajoutée lentement une solution d'anhydride trifluorométhanesulfonique (1,1 équivalents, 3 mol.L⁻¹) dans le dichlorométhane anhydre. Le milieu réactionnel est agité pendant une heure à 0° C, puis, après remontée à température ambiante, une solution aqueuse saturée de NaCl est ajoutée. La phase aqueuse est extraite deux fois au dichlorométhane. Les phases organiques sont jointes, séchées sur sulfate de sodium anhydre, filtrées et concentrées à sec. Le résidu brut obtenu est purifié par chromatographie sur colonne de silice, la phase mobile étant un gradient CH₂Cl₂/MeOH.

Les monosulfonamides suivants ont été préparés :

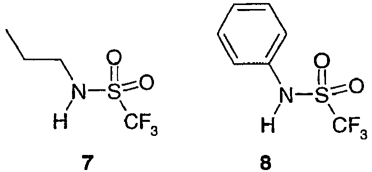

### Exemple 7 :

**RMN ¹H** (CDCl₃, 300 MHz) : δ 0,95-1,00 (t, ³J = 7,1 Hz, 3H, CH₃) ; 1,58-1,70 (m, 2H, CH₂) ; 3,25-3,30 (t, ³J = 7,4 Hz, 2H, CH₂) ; 4,89 (br s, 1H, NH) ppm ; ¹⁹F (CDCl₃, 280 MHz) : δ -77,4 ppm ; **SM (IE)** : 191 [M]⁺, 162 [M-C₂H₅]⁺.

### Exemple 8 :

**RMN ¹H** (CDCl₃, 300 MHz) : δ 7,19-7,28 (m, 3H, CH); 7,31-7,36 (m, 2H, CH); 6,63 (br s, 1H, NH) ppm ; **¹⁹F** (CDCl₃, 280 MHz) : δ -75,2 ppm ; **SM (IE)** : 225 [M]⁺.

### Préparation 2b : Préparation de bisulfonamides

Les bisulfonamides ont été préparés selon le mode opératoire suivant :

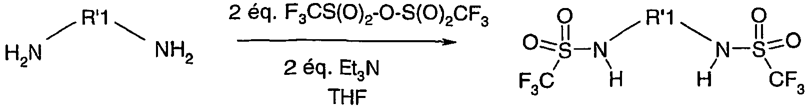

A un mélange de diamine (1 équivalent, 3 mol.L⁻¹) et de triéthylamine (2,1 équivalents) dans le dichlorométhane anhydre à 0° C, est ajoutée lentement une solution d'anhydride trifluorométhanesulfonique (2,1 équivalents, 5 mol.L⁻¹) dans le dichlorométhane anhydre. Le milieu réactionnel est agité une heure à froid, puis après remontée à température ambiante, une solution saturée de NaCl est ajoutée. La phase aqueuse est extraite deux fois au dichlorométhane. Les phases organiques sont jointes, séchées sur sulfate de sodium anhydre, filtrées et concentrées à sec. Le résidu brut obtenu est purifié par chromatographie sur colonne de silice, la phase mobile étant un gradient CH₂Cl₂/MeOH.

Les bisulfonamides suivants ont été préparés :

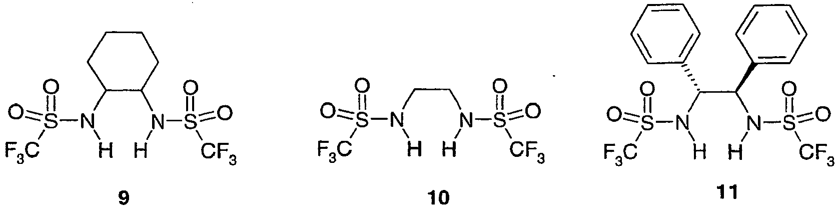

### Exemple 9 :

**RMN ¹H** ((CD₃)₂CO), 300 MHz) : δ 1,37-1,41 (m, 2H, CH) ; 1,67-1,71 (m, 2H, CH) ; 1,77-1,81 (m, 2H, CH) ; 2,11-2,15 (m, 2H, CH) ; 3,34 (m, 2H, CH) ; 7,98 (br s, 2H, NH) ppm ; **¹³C** ((CD₃)₂CO), 75 MHz) : δ 24,3 (CH₂) ; 33,2 (CH₂) ; 58,8 (CH); 115-128 (q, J_{CF} = 320,5 Hz, CF₃) ; **¹⁹F** ((CD₃)₂CO), 280 MHz): δ -77,5 ppm; **SM (IE)** : 378 [M]⁺, 245 FM-SO₂CF₃]⁺; **Point de fusion :** 184,5-185,5° C.

### Exemple 10 :

**RMN ¹H** (C₆D₆, 300 MHz) : δ 2,28 (s, 4H, CH₂) ; 4,00 (br s, 2H, NH) ; **¹³C** (C₆D₆, 75 MHz) : δ 43,4 (CH₂) ; 117-122 (q, J_{CF} = 321,5 Hz, CF₃) ; **¹⁹F** (C₆D₆, 280 MHz) : δ-77 ppm ; **SM (IC) :** 342 (M + NH₄⁺) ; **Analyse élémentaire :** *Calculé pour (C₄H₆F₆N₂O₄S₂)* C 14,8 %, H 1,87 %, N 8,64 %. *Mesuré* C 14,6 %, H 1,90 %, N 8,5 % 1^{er} essai et C 40,48 %, H 2,36 %, N 5,83 % 2^{ème} essai. **Point de fusion :** 115-116° C.

### Exemple 11 :

**RMN ¹H** (CDCl₃, 300 MHz) : δ 4,79 (s, 2H, CH) ; 5,87 (br s, 2H, NH) ; 6,97-7,00 (m, 4H, CH) ; 7,27 (m, 6H, CH) ppm ; **¹⁹F** (CDCl₃, 280 MHz) : δ-77,3 ppm ; **SM (IE)** : **Analyse élémentaire:** *Calculé pour (C₁₆H₁₄F₆N₂O₄S₂)* C 40,34 %, H 2,96 %, N 5,88 %. *Mesuré* C 40,43 %, H 2,36 %, N 5,82 % ; **Point de fusion :** 213-215° C.

### Préparation 3 : Préparation de bisulfonamides allkylés (exemples 12 à 13)

Les bisulfonamides alkylés ont été préparés selon le mode opératoire A ou B suivant :

### Protocole A:

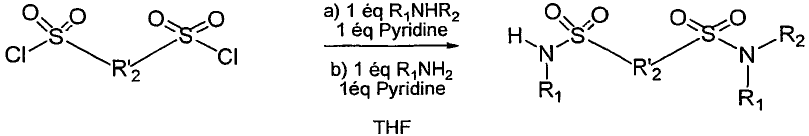

### Protocole B:

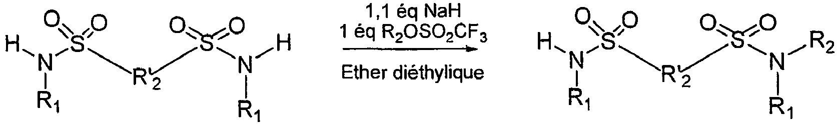

### Exemple 12 :

A un équivalent de pyridine (1,47 mL, 18 mmol) et un équivalent de N-méthyl aniline (1,97 mL, 18 mmol) en solution dans 50 ml de THF est introduit un équivalent de chlorure de 1,3-disulfonylbenzene (5 g, 18 mmol). Après deux heures d'agitation à température ambiante, un équivalent d'aniline (1,66 mL, 18 mmol) et un équivalent de pyridine (1,47 mL, 18 mmol) sont ajoutés. Le mélange est agité la nuit à température ambiante. Le solvant est évaporé sous vide. Le brut obtenu est dissout dans CH₂Cl₂), lavé avec une solution d'HCl 0,1N puis de l'eau, séché sur sulfate de sodium, filtré et évaporé. Le solide obtenu (mélange 0,33/1/0,33 nonméthylé / monométhylé / diméthylé) est purifié par chromatographie sur gel de silice (éluant CH₂Cl₂/MeOH 95/5) Le sulfonamide monométhylé 12 est obtenu sous forme d'un solide blanc avec un rendement de 50 %.

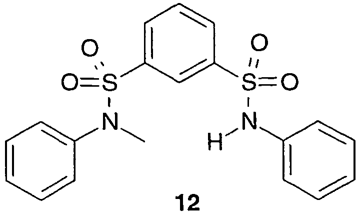

**RMN ¹H** (CDCl₃, 300 MHz) : δ 3,09 (s, 3H, CH₃) ; 6,84 (br s, 1H, NH) ; 6,94-6,98 (m, 2H, CH) ; 7,06 (m, 1H, CH) ; 7,09 (m, 1H, CH) ; 7,26-7,29 (m, 6H, CH) ; 7,49 (m, 1H, CH) ; 7,56-7,60 (m, 1H, CH) ; 7,91-7,94 (m, 1H, CH) ; 8,09 (t, 4J = 1,5 Hz, 1H, CH) ppm ; ¹³C (CDCl_{3,} 75,5 MHz) : δ 38,4 (CH₃) ; 122,1 (CH) ; 126,2 (CH) ; 126,4 (CH) ; 126,6 (CH) ; 127,9 (CH) ; 129,2 (CH) ; 129,6 (CH) ; 129,7 (CH) ; 131,2 (CH) ; 131,9 (CH) ; 135,7 (C) ; 138,0 (C) ; 140,2 (C) ; 140,7 (C) ppm ; **HRMS DCI (CH₄) :** Calculé pour 403,0786 (M+H+- C₁₉H₁₉N₂O₄S₂), Mesuré 403,0769 (-1,7;-4,3) ; **Point de fusion :** 162,0-162,6° C.

### Exemple 13 :

Sous atmosphère d'argon, 1,1 équivalents de NaH (206 mg, 8,6 mmol, huile enlevée par trois lavages au pentane) sont ajoutés à une solution d'un équivalent de disulfonamide 4 (2,75 g, 7,8 mmol) dans 100 mL d'éther diéthylique séché. Après 30 minutes d'agitation à tempéraure ambiante, un équivalent de triflate de méthyle (880 µL, 7,8 mmol) est ajouté. Le milieu réactionnel est agité à température ambiante sur la nuit puis le solvant est évaporé. Le brut est redissout dans CH₂Cl₂, lavé deux fois avec une solution d'HCl 1N, puis une solution saturée de sel, séché sur sulfate de sodium, filtré et reévaporé. Le solide obtenu (mélange 0,4/0,2/0,4 nonméthylé / monométhylé / diméthylé) est purifié par chromatographie sur gel de silice (éluant CH₂Cl₂/MeOH 95/5). Le sulfonamide 13 est isolé sous forme d'une poudre blanche avec un rendement de 12 %.

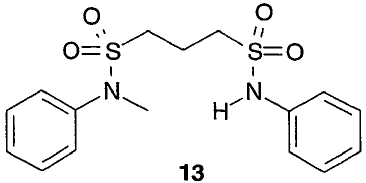

**RMN ¹H** (CDCl₃, 300 MHz) : δ 2,23 (m, 2H, CH₂) ; 3,13 (m, 4H, CH₂) ; 3,21 (s, 3H, CH₃) ; 7,08 (m, 1H, CH) ; 7,13-7,16 (m, 2H, CH) ; 7,19-7,24 (m, 3H, CH) ; 7,27 (m, 4H, CH) ; 7,34 (br s, 1H, NH) ppm ; ¹³C (CDCl₃, 75,5 MHz) : δ 18,0 (CH₂) ; 38,6 (CH₃) ; 47,1 (CH₂) ; 49,4 (CH₂) ; 120,8 (CH) ; 125,3 (CH) ; 126,7 (CH) ; 127,7 (CH) ; 129,5 (CH) ; 129,7 (CH) ; 136,6 (C) ; 140,9 (C) ppm ; **HRMS DCI (CH₄)** : Calculé pour 369,0943 (M+H+- C₁₆H₂₁N₂O₄S₂), Mesuré 369,0957 (1,4;-3,8) ; **Point de fusion :** 93,6-94,2° C.

### Préparation 4 : Utilisation des catalyseurs sulfonamides en polymérisation par ouverture de cycle (ROP) du lactide

Les catalyseurs préparés ont été testés en polymérisation par ouverture de cycle (ROP) du D,L-lactide (ou du L-lactide) en association avec différentes bases : 4-diméthylaminopyridine (DMAP), spartéine, diisopropyléthylamine (DIEA), N,N-diméthylcyclohexylamine (Me₂NCy), N,N,N"N"-tétraméthyl-1,2-cyclohexanediamine ((Me₂N)₂Cy).

Les polymères du lactide ont été préparés selon le mode opératoire suivant :

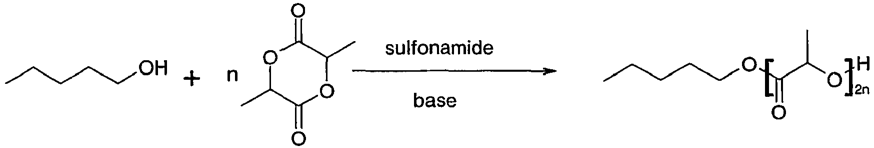

Pour toutes les polymérisations, dans un ballon schlenk sous argon, le lactide, le catalyseur et la base sont dissous dans un solvant anhydre (tel que le dichlorométhane, le toluène ou le tétrahydrofurane). Le pentanol est ajouté et le milieu réactionnel est agité à température ambiante. La conversion du lactide en polymère est suivie par prélèvements réguliers d'un échantillon de solution qui est concentré, redissous dans du CDCl₃, et contrôlé par RMN ¹H.

### Exemple 14 :

Dans un ballon schlenk sous argon, le lactide (500 mg, 10 éq, 3,47 mmol), le catalyseur de l'exemple 3 (134 mg, 1 éq, 0,347 mmol) et la DMAP (42 mg, 1 éq, 0,347 mmol) sont dissous dans 3,5 mL de dichlorométhane anhydre. Le pentanol (38 µL, 1 éq, 0,347 mmol) est ajouté et le milieu réactionnel est agité à 26° C. La conversion du lactide en polymère est suivi par prélèvements réguliers d'un aliquote de solution qui est concentré, redissous dans du CDCl₃, et contrôlé par RMN ¹H.

A conversion complète Mn = 1983 Mw = 2217 Ip = 1,12

Diverses synthèses de polymères courts ont permis de mettre en évidence la diversité des couples sulfonamide / base pouvant être utilisés, toutes conditions expérimentales égales par ailleurs (CH₂Cl₂ ; rapport molaire 5/1/1/1 de Lactide / Pentanol / Sulfonamide / Amine tertiaire).

| **Exemple** | Sulfonamide | Base | Temps | Conversion |
|---|---|---|---|---|
| **15** | 3 | DMAP | 5 h | 97 % |
| **16** | 3 | Me₂NCy | 5 h | 77 % |
| **17** | 3 | spartéine | 5 h | 73 % |
| **18** | 7 | DIEA | 5 h 15 | 84 % |
| **19** | 7 | spartéine | 5 h 15 | 91 % |
| **20** | 7 | DMAP | 5 h | 94 % |
| **21** | 7 | (Me₂N)₂Cy | 8 h | 80 % |
| **22** | 9 (R, R) | Me₂NCy | 5 h | 98 % |
| **23** | 10 | Me₂NCy | 6 h | 95 % |
| **24** | 11 | Me₂NCy | 72 h | 64 % |
| **25** | 5 | DMAP | 6 h | 84 % |
| **26** | 6 | DMAP | 5 h 15 | 76 % |

Des polymères de longueurs de chaînes différentes peuvent être synthétisés de manière contrôlée quel que soit le catalyseur utilisé.

Des oligomères (rapports molaires 5/1/1/1 de Lactide/Pentanol/Sulfonamide/DMAP ou 10/1/1/1 dans CH₂Cl₂) peuvent ainsi être obtenus de manière rapide et contrôlée.

| **Exemple** | M/I | Catalyseur | Temps | Conversion | DP_{RMN} |
|---|---|---|---|---|---|
| **27** | 5 | 3 | 3 h | 93 % | 4,8 |
| **28** | 10 | 1 | 24 h | 93 % | 9,9 |
| **29** | 10 | 2 | 40 h | 93 % | 9,1 |
| **30** | 10 | 3 | 8 h | 88 % | 8,7 |
| **31** | 10 | 4 | 8 h | 92 % | 8,8 |
| **32** | 10 | 12 | 8h | 67% | 5,8 |
| **33** | 10 | 13 | 7h30 | 55% | 5,2 |

M/I désigne le rapport molaire initial monomère/amorceur utilisé dans la polymérisation.

Le DP_{RMN} est le degré de polymérisation du polymère formé. Il est déterminé par intégration des signaux appropriés sur les spectres RMN ¹H.

Il est également possible de synthétiser des polymères de tailles plus importantes. Les rapports utilisés sont alors de 50/1/10/10 (Lactide/Pentanol/Sulfonamide/DMAP) ou de 100/1/10/10. Il est important de noter les indices de polydispersité reproductibles des polymères obtenus :

| **Exemple** | M/I | Catalyseur | Temps | Conversion | Mn | Mw | Ip |
|---|---|---|---|---|---|---|---|
| **34** | 10** | 3 | 8 h | 88 % | 1765 | 2067 | 1,17 |
| **35** | 50** | 3 | 24 h | 90 % | 7949 | 8413 | 1,06 |
| **36** | 50** | 5 | 24 h | 70 % | 5296 | 5729 | 1,08 |
| **37** | 100* | 3 | 87 h 30 | 94 % | 14830 | 16087 | 1,08 |
| **38** | 100** | 6 | 87 h 30 | 71 % | 9540 | 10116 | 1,06 |

| | | | | | | | |
|---|---|---|---|---|---|---|---|
| * L-lactide ** D,L-lactide | | | | | | | |

Les polymères synthétisés ont des masses correspondant au rapport monomère / amorceur utilisé, montrant un bon contrôle de la polymérisation. Les temps de réaction varient de 8 heures à trois jours, selon le catalyseur utilisé et le degré de polymérisation (DP) visé.

Les résultats expérimentaux ci-dessus montrent que les catalyseurs sulfonamides permettent la polymérisation du lactide. Par ailleurs les indices de polydispersité proches de 1 obtenus montrent que ces catalyseurs défavorisent les transestérifications.

## Revendications

1. Utilisation d'un sulfonamide en association avec une base comme système catalytique de (co)polymérisation de lactones par ouverture de cycle.

2. Utilisation selon la revendication 1 pour la (co)polymérisation de dilactones.

3. Utilisation selon la revendication 1 pour la copolymérisation du lactide et/ou du glycolide.

4. Utilisation selon l'une des revendications précédentes **caractérisée en ce que** ladite base est une amine tertiaire.

5. Utilisation selon l'une des revendications précédentes **caractérisée en ce que** ladite base est une amine tertiaire choisie parmi :
- diisopropyléthylamine ;
- spartéine ;
- N,N-diméthylcyclohexylamine ;
- N,N,N"N"-tétraméthyl-1,2-cyclohexanediamine ; et
- 4-diméthylaminopyridine.

6. Utilisation selon l'une des revendications précédentes **caractérisée en ce que** ledit sulfonamide est un monosulfonamide.

7. Utilisation selon l'une des revendications précédentes **caractérisée en ce que** ledit sulfonamide est un monosulfonamide de formule générale **(I)** : 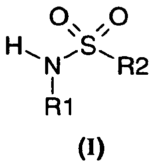 sous forme racémique, d'énantiomère ou toute combinaison de ces formes, dans laquelle :
R1 et R2 représentent indépendamment un radical alkyle, haloalkyle ou aryle éventuellement substitué.

8. Utilisation selon la revendication 7 **caractérisée en ce que** R1 et R2 représentent indépendamment un radical phényle, alkyle ou haloalkyle.

9. Utilisation selon l'une des revendications précédentes **caractérisées en ce que** le sulfonamide est un composé choisi parmi :

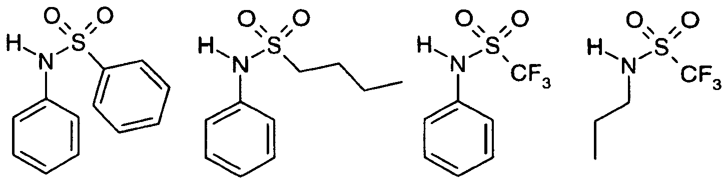

10. Utilisation selon l'une des revendications 1 à 5 **caractérisée en ce que** ledit sulfonamide est un bisulfonamide.

11. Utilisation selon la revendication 10 **caractérisée en ce que** le sulfonamide est un bisulfonamide de formule générale **(IIa)** ou **(IIb)** 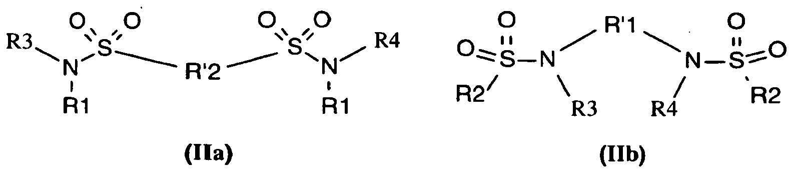 sous forme racémique, d'énantiomère ou toute combinaison de ces formes, dans laquelle :
R1 et R2 représentent un radical alkyle, haloalkyle ou aryle éventuellement substitué ;
R'1 et R'2 représentent un radical arylène, alkylène ou cycloalkylène, tous ces radicaux étant éventuellement substitués ;
R3 et R4 représentent indépendamment un atome d'hydrogène ou un radical alkyle.

12. Utilisation selon la revendication 11 **caractérisée en ce que** R1 et R2 représentent un radical phényle éventuellement substitué, alkyle ou haloalkyle ; R'1 représente un radical cycloalkylène, alkylène éventuellement substitué par un radical phényle ; R'2 représente un radical alkylène ou phénylène.

13. Utilisation selon l'une des revendications 11 à 12 à **caractérisée en ce que** R1 et R2 représentent un radical alkyle, trifluorométhyle ou phényle éventuellement substitué par méthyle ou trifluorométhyle.

14. Utilisation selon la revendication 11 **caractérisée en ce que** le sulfonamide est un composé choisi parmi :

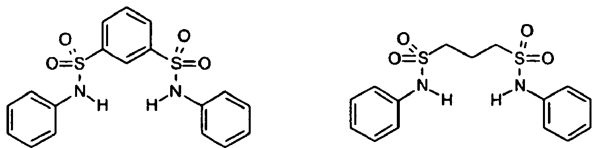

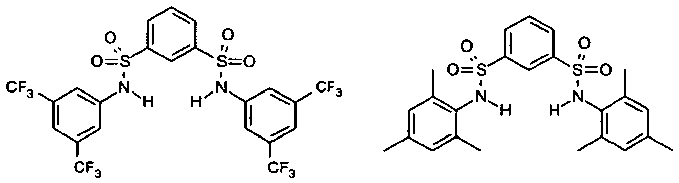

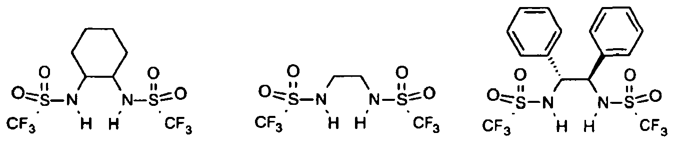

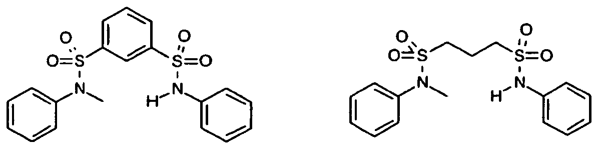

15. Utilisation selon l'une des revendications 11 à 14 **caractérisée en ce que** le sulfonamide est un composé choisi parmi :

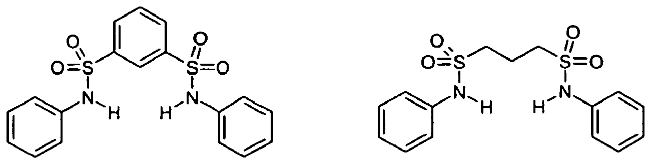

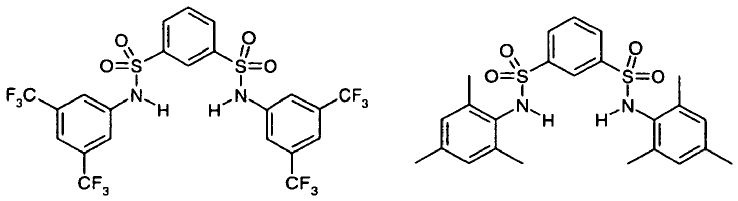

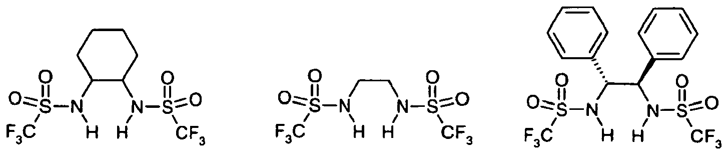

16. Utilisation selon l'une des revendications précédentes **caractérisée en ce que** la base est la 4-diméthylaminopyridine.

17. Composés de structure :

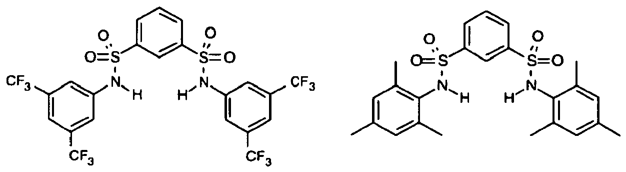

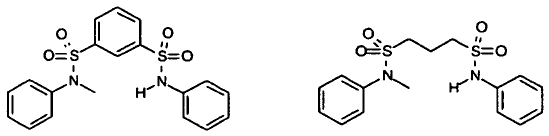

18. Procédé de (co)polymérisation par ouverture de cycle **caractérisé en ce qu'**il fait intervenir l'utilisation d'un sulfonamide en association avec une base comme catalyseur, selon l'une des revendications 1 à 16.

## Patentansprüche

1. Verwendung eines Sulfonamids in Verbindung mit einer Base als katalytisches System zur (Co)Polymerisation von Lactonen durch Ringöffnung.

2. Verwendung nach Anspruch 1 für die (Co)Polymerisation von Dilactonen.

3. Verwendung nach Anspruch 1 für die Copolymerisation von Lactid und/oder Glycolid.

4. Verwendung nach einem der vorangehenden Ansprüche, **dadurch gekennzeichnet, dass** die Base ein tertiäres Amin ist.

5. Verwendung nach einem der vorangehenden Ansprüche, **dadurch gekennzeichnet, dass** die Base ein tertiäres Amin ist, das unter:
- Düsopropylethylamin;
- Spartein;
- N,N-Dimethylcyclohexylamin;
- N,N,N",N"-Tetramethyl-1,2-cyclohexandiamin und
- 4-Dimethylaminopyridin
ausgewählt ist.

6. Verwendung nach einem der vorangehenden Ansprüche, **dadurch gekennzeichnet, dass** das Sulfonamid ein Monosulfonamid ist.

7. Verwendung nach einem der vorangehenden Ansprüche, **dadurch gekennzeichnet, dass** das Sulfonamid ein Monosulfonamid der allgemeinen Formel (I): 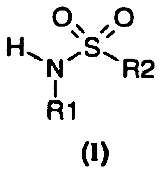 in racemischer Form, Enantiomerform oder allen Kombinationen dieser Formen ist, in der:
R1 und R2 unabhängig einen Alkyl-, Halogenalkyl- oder Arylrest, gegebenenfalls substituiert, darstellen.

8. Verwendung nach Anspruch 7, **dadurch gekennzeichnet, dass** R1 und R2 unabhängig einen Phenyl-, Alkyl- oder Halogenalkylrest darstellen.

9. Verwendung nach einem der vorangehenden Ansprüche, **dadurch gekennzeichnet, dass** das Sulfonamid eine Verbindung ist, die aus 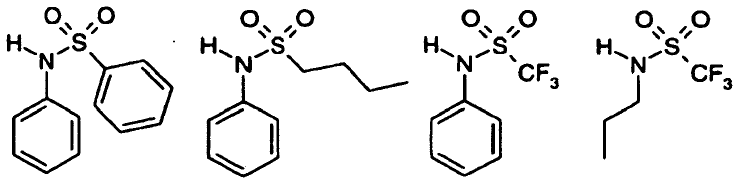 ausgewählt ist.

10. Verwendung nach einem der Ansprüche 1 bis 5, **dadurch gekennzeichnet, dass** das Sulfonamid ein Bisulfonamid ist.

11. Verwendung nach Anspruch 10, **dadurch gekennzeichnet, dass** das Sulfonamid ein Bisulfonamid der allgemeinen Formel (IIa) oder (IIb) 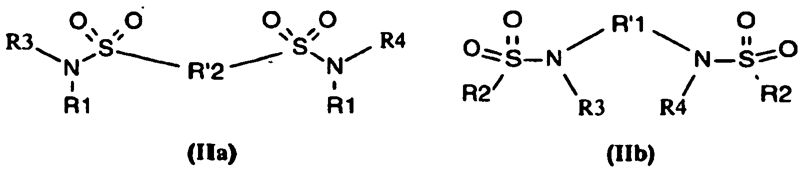 in racemischer Form, Enantiomerform oder allen Kombinationen dieser Formen ist, worin:
R1 und R2 einen Alkyl-, Halogenalkyl- oder Arylrest, gegebenenfalls substituiert, darstellen;
R'1 und R'2 einen Arylen-, Alkylen- oder Cycloalkylenrest darstellen, wobei alle diese Reste gegebenenfalls substituiert sind;
R3 und R4 unabhängig ein Wasserstoffatom oder einen Alkylrest darstellen.

12. Verwendung nach Anspruch 11, **dadurch gekennzeichnet, dass** R1 und R2 einen gegebenenfalls substituierten Phenylrest, Alkyl- oder Halogenalkylrest darstellen; R'1 einen Cycloalkylen-, Alkylenrest, gegebenenfalls substituiert mit einem Phenylrest, darstellt; R'2 einen Alkylen- oder Phenylenrest darstellt.

13. Verwendung nach einem der Ansprüche 11 bis 12, **dadurch gekennzeichnet, dass** R1 und R2 einen Alkyl-, Trifluormethyl- oder Phenylrest, gegebenenfalls substituiert mit Methyl oder Trifluormethyl, darstellen.

14. Verwendung nach Anspruch 11, **dadurch gekennzeichnet, dass** das Sulfonamid eine Verbindung ist, die aus: 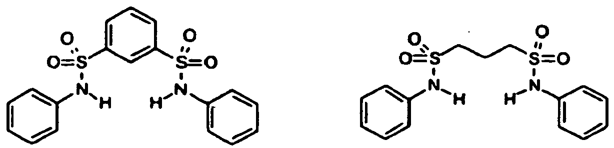 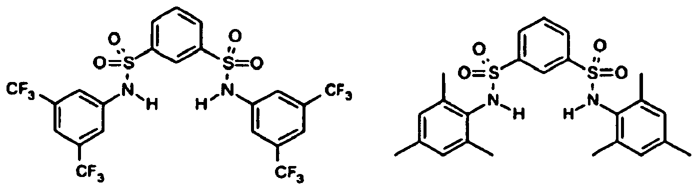 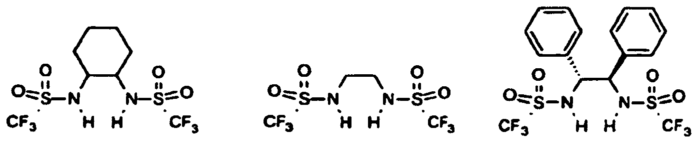 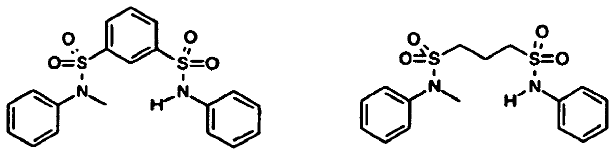 ausgewählt ist.

15. Verwendung nach einem der Ansprüche 11 bis 14, **dadurch gekennzeichnet, dass** das Sulfonamid eine Verbindung ist, die aus: 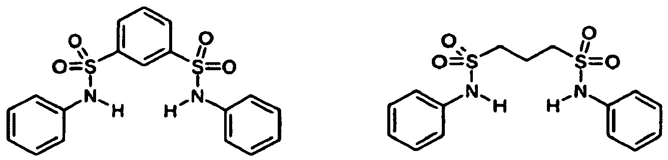 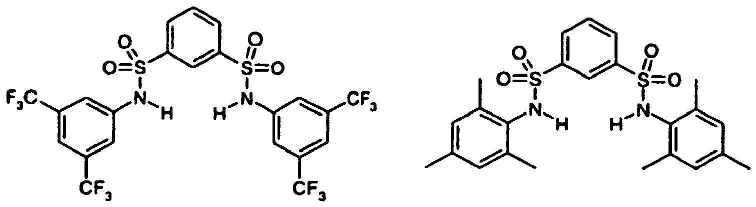 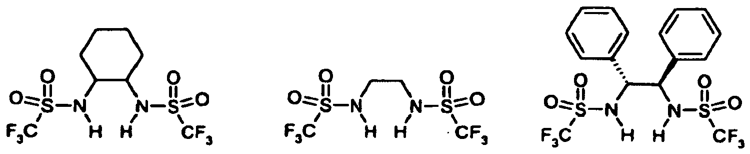 ausgewählt ist.

16. Verwendung nach einem der vorangehenden Ansprüche, **dadurch gekennzeichnet, dass** die Base 4-Dimethylaminopyridin ist.

17. Verbindungen der Struktur:

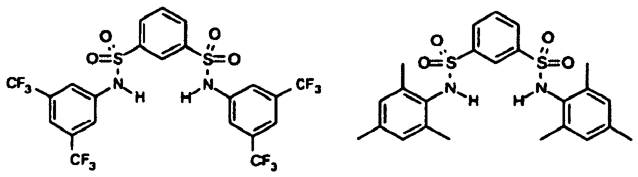

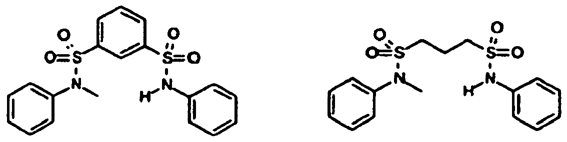

18. Verfahren zur (Co)Polymerisation durch Ringöffnung, **dadurch gekennzeichnet, dass** es die Verwendung eines Sulfonamids in Verbindung mit einer Base als Katalysator nach einem der Ansprüche 1 bis 16 umfasst.

## Claims

1. Use of a sulphonamide in combination with a base as a catalytic system for the ring-opening (co)polymerization of lactones.

2. Use according to claim 1 for the (co)polymerization of dilactones.

3. Use according to claim 1 for the copolymerization of lactide and/or glycolide.

4. Use according to one of the previous claims **characterized in that** said base is a tertiary amine.

5. Use according to one of the previous claims **characterized in that** said base is a tertiary amine chosen from:
- diisopropylethylamine;
- sparteine;
- N,N-dimethylcyclohexylamine;
- N,N,N"N"-tetramethyl-1,2-cyclohexanediamine; and
- 4-dimethylaminopyridine.

6. Use according to one of the previous claims **characterized in that** said sulphonamide is a monosulphonamide.

7. Use according to one of the previous claims **characterized in that** said sulphonamide is a monosulphonamide of general formula **(I)**: 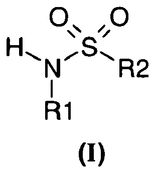 in racemic, or enantiomeric form or any combination of these forms, in which:
R1 and R2 represent independently an alkyl, haloalkyl or aryl radical, optionally substituted.

8. Use according to claim 7 **characterized in that** R1 and R2 represent independently a phenyl, alkyl or haloalkyl radical.

9. Use according to one of the previous claims **characterized in that** the sulphonamide is a compound chosen from:

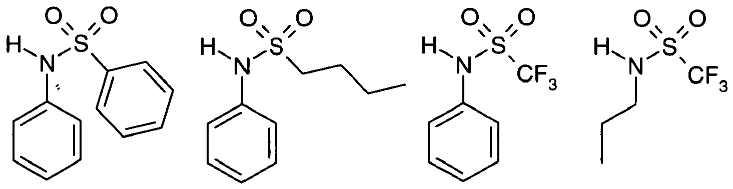

10. Use according to one of claims 1 to 5 **characterized in that** said sulphonamide is a bisulphonamide.

11. Use according to claim 10 **characterized in that** the sulphonamide is a bisulphonamide of general formula **(IIa)** or **(IIb)** 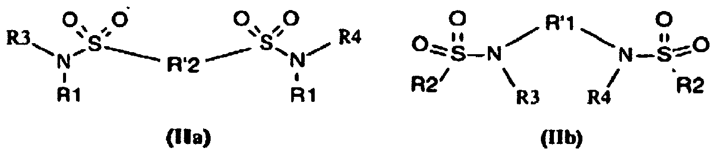 in racemic, or enantiomeric form or any combination of these forms, in which:
R1 and R2 represent an alkyl, haloalkyl or aryl radical, optionally substituted;
R'1 and R'2 represent an arylene, alkylene or cycloalkylene radical, all these radicals being optionally substituted;
R3 and R4 represent independently a hydrogen atom or an alkyl radical.

12. Use according to claim 11 **characterized in that** R1 and R2 represent an optionally substituted phenyl radical, alkyl or haloalkyl; R'1 represents a cycloalkylene, alkylene radical optionally substituted by a phenyl radical; R'2 represents an alkylene or phenylene radical.

13. Use according to one of claims 11 to 12 **characterized in that** R1 and R2 represent an alkyl, trifluoromethyl or phenyl radical optionally substituted by methyl or trifluoromethyl.

14. Use according to claim 11 **characterized in that** the sulphonamide is a compound chosen from:

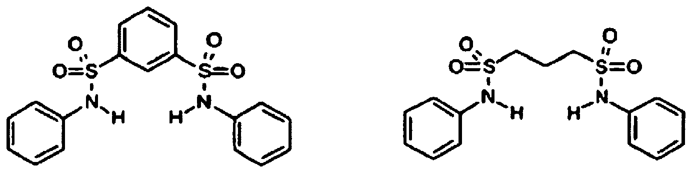

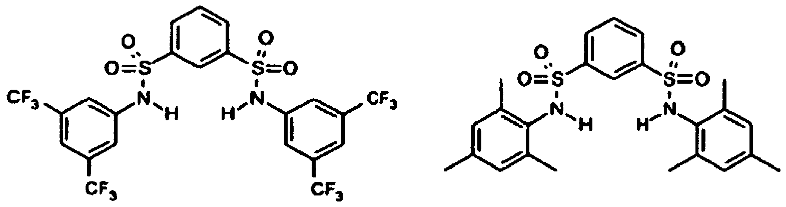

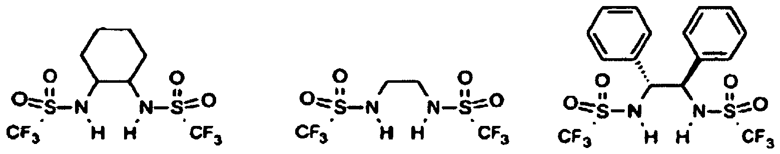

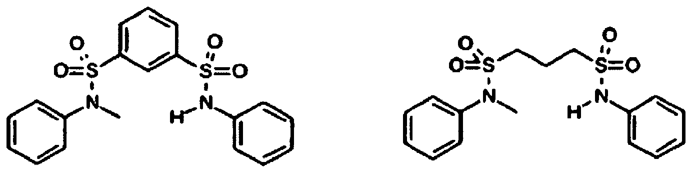

15. Use according to one of claims 11 to 14 **characterized in that** the sulphonamide is a compound chosen from:

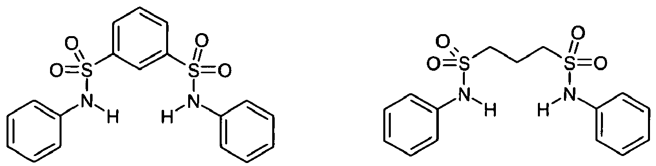

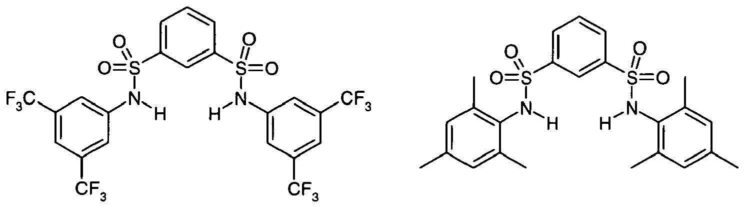

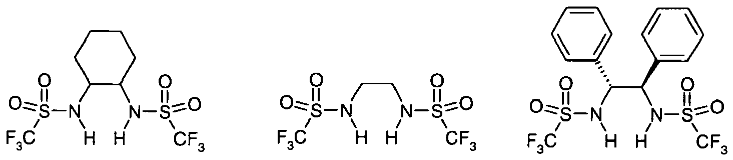

16. Use according to one of the previous claims **characterized in that** the base is 4-dimethylaminopyridine.

17. Compounds of structure:

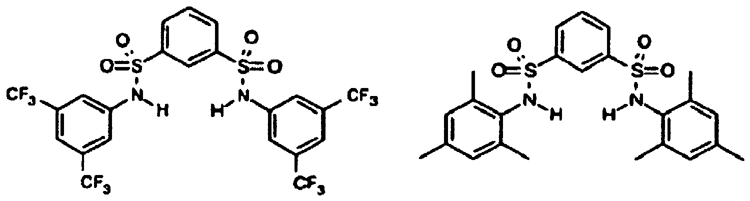

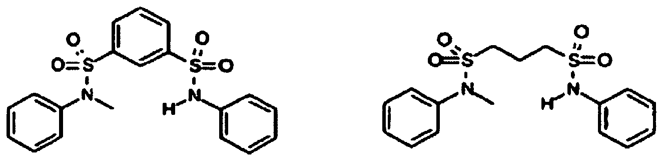

18. Process for ring-opening (co)polymerization **characterized in that** it involves the use of a sulphonamide in combination with a base as a catalyst, according to one of claims 1 to 16.
